## Europäisches Patentamt
## European Patent Office
## Office européen des brevets

(19)

(11) Veröffentlichungsnummer : **0 029 513**
**B1**

(12) **EUROPÄISCHE PATENTSCHRIFT**

(45) Veröffentlichungstag der Patentschrift :
16.02.83

(21) Anmeldenummer : 80106571.5

(22) Anmeldetag : 25.10.80

(51) Int. Cl.³ : **C 07 C   7/152**, C 07 C  11/04//
C07C11/06, C07C11/02

(54) Verfahren zur Abtrennung von Olefinen aus Olefine enthaltenden Gasen.

(30) Priorität : 02.11.79 DE 2944151

(43) Veröffentlichungstag der Anmeldung :
03.06.81 Patentblatt 81/22

(45) Bekanntmachung des Hinweises auf die Patenterteilung : 16.02.83 Patentblatt 83/07

(84) Benannte Vertragsstaaten :
BE DE FR GB IT NL

(56) Entgegenhaltungen :
US A 3 960 976
Chemical Abstracts Band 79, Nr. 10, 1973 Columbus, Ohio, USA J.V. CROOKES et al. : « Competitive Interactions in the complexing of ethylene
with silver (I) salt solutions » Seiten 343 bis 344 ;
Spalten 2 bzw. 1, Abstracts Nr. 58300v
J.V. CROOKES et al. : « Competitive Interactions
in the complexing of ethylene with silver (I) salt
solutions » J. Chem. Soc. Daitnr T 1973 (12) p.
1241-47.

(73) Patentinhaber : EC ERDÖLCHEMIE GMBH
Postfach 75 2002
D-5000 Köln 71 (DE)

(72) Erfinder : Alter, Eduard, Dr.
Ostpreussenallee 10
D-4047 Dormagen 1 (DE)
Erfinder : Bruns, Ludwig, Dr.
Am Kronenpützchen 26
D-4047 Dormagen 1 (DE)

(74) Vertreter : Dill, Erwin, Dr. et al
c/o BAYER AG Zentralbereich Patente Marken und
Lizenzen Bayerwerk
D-5090 Leverkusen 1 (DE)

# 0 029 513

## Verfahren zur Abtrennung von Olefinen aus Olefine enthaltenden Gasen

Die vorliegende Erfindung betrifft ein Verfahren, Olefine aus Olefine enthaltenden Gasströmen mit Hilfe einer wäßrigen Silbersalzlösung abzutrenne und aus der Lösung in reiner Form zurückzugewinnen.

In der Literatur ist seit langem bekannt, daß eine Reihe bestimmter Schwermetallionen, beispielsweise Platin(II)-, Palladium(II)-, Quecksilber(II)-, Kupfer(I)- und Silber(I)-Ionen, Komplexe mit einer Vielzahl von Olefinen bilden. Besonders die drei letztgenannten Metallionen besitzen technisches Interesse hinsichtlich der Abtrennung von ungesättigten Kohlenwasserstoffen aus olefinhaltigen Gemischen.

Quecksilber(II)-Salzlösungen scheiden jedoch für technische Zwecke aus, da einmal der Quecksilber-Olefin-Komplex eine sehr große Stabilität besitzt und die Olefine erst durch Zusatz von Alkali oder Säure bei stark erhöhter Temperatur freigesetzt werden können und zum anderen die hohe Giftigkeit des Quecksilbers eine technische Anwendung fraglich erscheinen läßt.

Bei den Kupfer(I)-Salzen besteht die Schwierigkeit der leichten Oxidierbarkeit des Cu(I) zu Cu(II), die im technischen Betrieb wegen der Notwendigkeit des Sauerstoffausschlußes zu erheblichen Schwierigkeiten führen kann.

Demgegenüber erscheint die Verwendung von Silbertetrafluoroborat und Silberhexafluorosilikat zur Abtrennung von Olefinen günstiger, da die hieraus gebildeten Silber-Olefin-Komplexe durch Auskochen der Lösung weitgehend wieder in Metallsalz und Olefin zerlegt werden können (DE-AS 1027 658) und die Silberionen relativ ungiftig sind. Die Löslichkeit der Olefine wird von der Silberionen-Konzentration und vom Olefin-Partialdruck bestimmt. Mit steigendem Silbergehalt und mit Druckerhöhung nimmt die Löslichkeit der Olefine in Silbersalzlösungen zu. Die in der angegebenen DE-AS genannten Silbersalzlösungen haben jedoch die folgenden Nachteile :

Silbertetrafluoroborat und Silberhexafluorosilicat sind sehr empfindlich gegen Hydrolyse, die in wäßriger Lösung nur mit einem Überschuß von Fluoroborsäure, bzw. Flußsäure zurückgedrängt werden kann. Die freie vorhandene Säure in der Lösung stellt bei einer technischen Durchführung erhebliche Anforderungen an die Apparatematerialien. So müssen beispielsweise Materialien wie Gold, Platin oder Graphit eingesetzt werden.

Weiterhin lassen sich beim Einsatz von Silbertetrafluoroborat Silbersalzlösungen von beträchtlicher Konzentration herstellen, die erhebliche Mengen an Olefinen aufnehmen, beim anschließenden Auskochen, beispielsweise bei 90 bis 100 °C, die Olefine jedoch nicht quantitativ freisetzen. Um bei der Desorption möglichst viel Olefin freizusetzen, arbeitet man daher im Vakuum, was einen weiteren technischen Aufwand bedeutet, da beispielsweise das im Vakuum desorbierte Olefingas einer nachgeschalteten Druckerhöhung unterzogen werden muß. Weiterhin werden bei diesen Bedingungen in der Desorptionsstufe erhebliche Mengen an freier Säure aus der Lösung in die Gasphase übergeführt, so daß der anfallende Olefinstrom einer nachgeschalteten alkalischen Säurewäsche unterzozen werden muß. Dies bedeutet einen Verlust an Säure und einen Aufwand an Chemikalien zu deren Neutralisation. Die Säurewäsche des geschilderten Verfahrens muß weiterhin für bestimmte Prozesse, beispielsweise für den Einsatz des desorbierten Ethylens in der Ethylenoxid-Herstellung besonders aufwendig durchgeführt werden, da bei der Ethylenoxid-Herstellung unkontrollierbare Mengen an Halogenverbindungen als Katalysatorgifte wirken. Außerdem wird für die technische Durchführung der Olefinabsorption mittels Silbertetrafluoroboratlösung empfohlen, einen Olefinpartialdruck von 2 bar nicht zu überschreiten (Erdöl und Kohle, Erdgas, Petrochemie, *16*, 551 ff (1963). Der Grund hierfür ist die Neigung der Olefine in Gegenwart von Tetrafluoroborsäure, bei Drucken über 2 bar und bei Temperaturen von 10-40 °C Oligomere zu bilden, die es erforderlich machen, die Silbersalzlösung nach einer gewissen Betriebszeit auszuwechseln.

Die geschilderten Nachteile haben es bisher offenbar verhindert, daß die vorgeschlagenen Verfahren im technischen Maßstab angewendet werden.

Aus J. Chem. Soc., Dalton Trans. 1973 (12), Seite 1241-1247 sind vergleichende Messungen der Absorptionskonstanten von Ethylen an Silberperchlorat, -trifluoracetat und -nitrat bekannt. Diese Messungen wurden in einem statischen System durchgeführt und enthalten keine Aussage über die Desorption und die Anwendung in einem kontinuierlichen Verfahren. Die Untersuchungen wurden mit Rein-Ethylen durchgeführt und enthalten demnach keine Aussagen über selektive Absorption.

In US 3 960 976 wird die Bildung von Olefin-Silbertrifluoracetat-Komplexen in wasserfreiem Medium beschrieben. Diese Komplexe werden nach ihrer Isolierung durch Behandlung mit Wasser in das Olefin und wäßrige $CF_3COOAg$-Lösung zersetzt. Zur Wiederverwendung muß die $CF_3COOAg$-Lösung auf trockenes $CF_3COOAg$ Aufgearbeitet werden.

Es wurde nun ein Verfahren zur Abtrennung von Olefinen aus Olefine enthaltenden Gasen durch Absorption der Olefine in einer wäßrigen Lösung eines Silbersalzes und Rückgewinnung der Olefine in reiner Form durch Desorption aus der Silbersalzlösung gefunden, das durch gekennzeichnet ist, daß man als Silbersalz Silbertrifluoracetat verwendet und die Absorption der Olefine in an sich bekannter Weise bei höherem Druck und/oder niedrigerer Temperatur als die Desorption durchführt.

Die wäßrige Lösung des Silbertrifluoracetats kann beispielsweise mit einer Konzentration von 400 bis 720 g $CF_3COOAg/1$, bevorzugt 500 bis 660 g/l, im erfindungsgemäßen Verfahren eingesetzt werden.

Als Olefine für das erfindungsgemäße Verfahren seien beispielsweise geradkettige oder verzweigte

2

Monomere für die Vinylpolymerisation mit einer oder mehreren, bevorzugt einer, Doppelbindung(en) und 2 bis 6, bevorzugt 4, besonders bevorzugt 2 oder 3 Kohlenstoffatomen genannt. Beispiels hierfür sind : Ethylen, Propylen, n-Buten-1, n-Buten-2, i-Buten, Butadien, die isomeren Pentene, die isomeren Hexene, Acrylnitril, Vinylacetat, Acrylsäure, Acrylsäureester, Methacrylsäure und Methacrylsäureester. Bevorzugt werden Ethylen und Propylen, besonders bevorzugt Ethylen, abgetrennt.

Die olefinhaltigen Gase können neben einem oder mehreren der genannten Olefine noch gesättigte Kohlenwasserstoffe, Kohlendioxid, Kohlenmonoxid, Sauerstoff, Wasserstoff, Stickstoff oder Edelgase — beispielsweise Argon — enthalten. Die genannten Stoffe können in stark wechselnder Menge im olefinhaltigen Gas vorhanden sein, ohne daß dadurch die Durchführung des erfindungsgemäßen Verfahrens beeinträchtigt wird. Die Menge der Olefine im olefinhaltigen Abgas beeinträchtigt die Durchführbarkeit des erfindungsgemäßen Verfahrens ebenfalls nicht. Beispielsweise sei ein Gehalt von etwa 5 bis über 95 Vol.-% Olefine im olefinhaltigen Gas genannt. Ganz allgemein ist die Olefinmenge im olefinhaltigen Gas von dem vorgeschalteten Prozeß abhängig, in dem das olefinhaltige Gas angällt.

Als Beispiel sei die Anwendung des erfindungsgemäßen Verfahrens auf die Abtrennung von Ethylen aus einem Abgas der Ethylenoxid-Herstellung genannt. Als typische Zusammensetzung eines solchen Abgases sei für den Fall, daß die Ethylenoxid-Herstellung mit Methan als Inertgas-Verdünnung durchgeführt wird, die folfende genannt :

35-55 Vol.-% Methan, 20-35 Vol.-% Ethylen, 4-6 Vol.-% Kohlendioxid, 3-6 Vol.-% Sauerstoff, 0,1 bis 10 Vol.-% Argon und 0,1-10 Vol.-% Stickstoff. Für den Fall, daß bei der Ethylenoxid-Herstellung Stickstoff anstelle von Methan als Inertgas-Verdünnung benutzt wird, kann der angegebene Gehalt an Methan auf 0 absinken, während der Stickstoffgehalt Werte von 35-60 Vol.-% annehmen kann.

Als Temperatur für die Absorption der Olefine sei beispielsweise ein Bereich von etwa 10 bis etwa 60 °C, bevorzugt etwa 15 bis etwa 30 °C, genannt. Als Temperatur für die Desorption sei beispielsweise ein Temperaturbereich von etwa 15 bis etwa 100 °C, bevorzugt etwa 50 bis etwa 90 °C, genannt.

Als Druck für die Absorption der Olefine sei Normaldruck oder Überdruck, beispielsweise im Bereich von 1 bis 25 bar, bevorzugt im Bereich von 1 bis 15 bar, genannt. Als Druck für die Desorption der Olefine sei Unterdruck, Normaldruck oder geringer Überdruck, beispielsweise im Bereich von 0,05 bis 4 bar, bevorzugt 1 bis 4, genannt. Wegen der Einfachheit des apparativen Aufwandes kann die Desorption der Olefine, besonders bevorzugt bei Normaldruck vorgenommen werden.

Die Absorption und die Desorption der Olefine im erfindungsgemäßen Verfahren werden mit verschiedenen Druck-Temperatur-Zuständen betrieben. So kann beispielsweise die Absorption der Olefine bei höherem Druck als die Desorption durchgeführt werden. Die Absorption der Olefine kann jedoch auch bei niedrigerer Temperatur als die Desorption durchgeführt werden. Schließlich können beide Maßnahmen in Kombination angewendet werden. Zur Durchführung dieser Maßnahmen werden an oder zwischen den Absorptions- und Desorptionsapparaten Einrichtungen angebracht, die die erforderlichen Veränderungen des Druckes und/oder der Temperatur ermöglichen, beispielsweise Pumpen bzw. Entspannungsventile und/oder Kühl-bzw. Heizeinrichtungen.

Die Kombination der Druck- und Temperaturdifferenzen in der Absorptions- bzw. Desorptionsstufe ist die bevorzugte Durchführung des erfindungsgemäßen Verfahrens. Hierbei werden für die Absorption eine Temperatur von etwa 15 bis etwa 30 °C und ein Überdruck und für die Desorption eine Temperatur von etwa 50 bis etwa 90 °C und einen Druck von mindestens Normaldruck eingestellt. In einer besonders bevorzugten Ausführungsform wird in der Absorptionsstufe ein Druck von mindestens 2 bar eingestellt, wobei der Druck für die Absorption höher ist als der Druck für die Desorption.

Das erfindungsgemäße Verfahren kann diskontinuierlich oder kontinuierlich durchgeführt werden. Bevorzugt ist die kontinuierliche Durchführung. Hierbei wird eine wäßrige Lösung von Silbertrifluoracetat kontinuierlich durch einen Absorptionsapparat gepumpt, wo sie sich mit den Olefinen aus dem olefinhaltigen Gas belädt, anschließend in einen Desorptionsapparat gepumpt, wo die Olefine in reiner Form zurückgewonnen werden, und danach wieder in die Absorptionsstufe zurückgepumpt. Als Absorptionsapparate kommen dem Fachmann bekannte Apparate mit geeigneten Einbauten in Frage, die es gestatten, die olefinhaltigen Gase und die Silbertrifluoracetatlösung in einen innigen Kontakt zu bringen. Als solche Einbauten seien beispielsweise genannt : Füllkörper-Packungen, eingebaute Böden, Siebe oder Umlenkbleche oder Düsen zur feinen Verteilung der Silbertrifluoracetatlösung. Als Desorptionsapparate kommen solche dem Fachmann bekannten Apparate mit Einbauten in Frage, die es gestatten, die mit den Olefinen beladene Silbertrifluoracetatlösung zur Erzielung einer großen Oberfläche fein zu verteilen. Beispielsweise können solche Apparate verwendet werden, wie sie für die Absorption beschrieben worden sind. Zur Unterstützung der Desorption der Olefine kann es nützlich sein, der mit den Olefinen beladenen Silbertrifluoracetatlösung geeignete Gase entgegenzublasen. Als solche seien beispielsweise Stickstoff, Methan oder Wasserdampf genannt.

Das erfindungsgemäße Verfahren ermöglicht die selektive Abtrennung von Olefinen aus einem olefinhaltigen Gas. Es können etwa 85 bis 95 % der im olefinhaltigen Gas enthaltenen Olefine zurückgewonnen werden. Die zurückgewonnenen Olefine haben eine Reinheit von mehr als 98 Vol.-%.

Es ist überraschend, daß das erfindungsgemäße Verfahren mit einem nur etwa halb so großen Silbergehalt in der Absorptionslösung, verglichen mit dem Stand der Technik, die gleiche Absorptionsleistung zeigt. Es ist ferner überraschend, daß im erfindungsgemäßen Verfahren keine Umsetzung der absorbierten Olefine zu Oligomeren erfolgt, so daß die Absorptionslösung über praktisch unbegrenzte

0 029 513

Zeit gebrauchsfähig bleibt.

Im erfindungsgemäßen Verfahren wird eine säurefreie Absorptionslösung eingesetzt, so daß eine Erleichterung bei der Auswahl der Materialien für die Apparate gegeben ist. Weiterhin geht bei der Desorption der Olefine keine freie Säure in die Reinolefine, so daß auf eine nachgeschaltete Säurewäsche der Reinolefine verzichtet werden kann. Obwohl die Desorption erfindungsgemäß auch unter Anwendung von Unterdruck erfolgen kann, ist sie bereits bei Normaldruck oder einem leichten Überdruck, beispielsweise bis etwa 2 bar, und einer Temperatur von etwa 80 bis etwa 90 °C quantitativ erfolgt, so daß ein technisch aufwendiges Arbeiten im Vakuum nicht erforderlich ist. Im Gegensatz zu den Angaben des Standes der Technik kann das erfindungsgemäße Verfahren auch bei Drucken über 2 bar angewendet werden, ohne daß nachteilige Erscheinungen, wie beispielsweise eine Oligomerisierung der absorbierten Olefine, beobachtet werden.

Beispiele

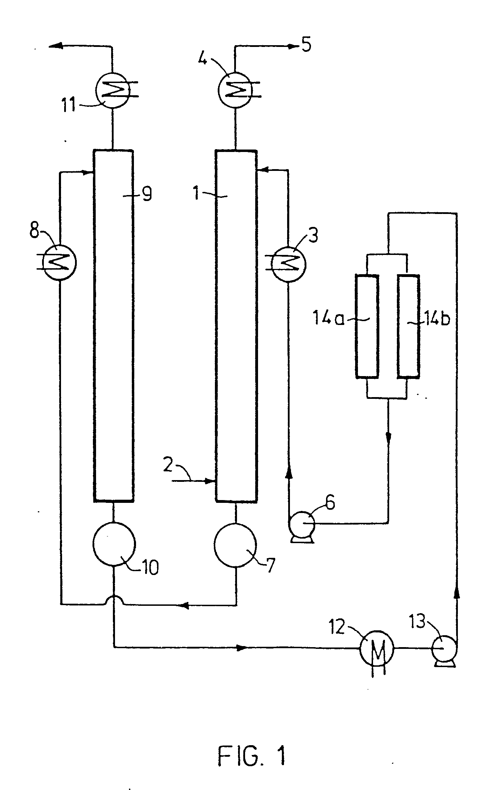

Die folgenden Beispiele werden in einer Kreislaufapparatur, wie sie in Abbildung 1 gezeigt wird, kontinuierlich durchgeführt.

Das olefinhaltige Gas tritt bei (2) in den Absorber (1), wo das Olefin von der im Gegenstrom geführten Silbertrifluoracetatlösung absorbiert wird. Das Abgas wird zur Wasserabscheidung über den Kühler (4) zum Auslaß (5) geleitet. Die mit Olefin beladene Absorptionslösung gelangt über eine Vorlage (7) und einen Vorwärmer (8) in den Desorber (9). Das desorbierte Reinolefin wird zur Wasserabscheidung über den Kühler (11) der Wiederverwendung zugeführt. Die Absorptionslösung wird über die Vorlage (10), den Kühler (12) und die Pumpe (13) in die Vorratsbehälter (14a, 14b) gebracht, von wo sie über die Pumpe (6) und den Kühler (3) wieder in den Absorber (1) geführt wird.

Beispiel 1

Verwendet wird eine Silbertrifluoracetat-Lösung mit 600 g $CF_3COOAg/l$. Die Umlaufmenge beträgt 1,2 l/h. Der Absorber wird beaufschlagt mit 60 l/h eines äthylenhaltigen Abgasstromes.

In den beiden folgenden Tabellen sind die Versuchsdaten zusammengefaßt.

| Absorber | $p$ = 1,27 bar ; T = 20 °C |
| Desorber | $p$ = 1,0  bar ; T = 90 °C |
| Gas zum Absorber | 60 Nl/h |
| Umlauf-Lösung | 1,2 l/h |

|  | Eingang | | Abgas | | Reinäthylen | |
|---|---|---|---|---|---|---|
|  | Nl/h | Vol.-% | Nl/h | Vol.-% | Nl/h | Vol.-% |
| $CH_4$ | 32,0 | 53,4 | 32,0 | 77,2 | * | 0,1 |
| $C_2H_4$ | 19,2 | 32,0 | 0,8 | 1,9 | 18,4 | 99,0 |
| $CO_2$ | 2,9 | 4,8 | 2,8 | 6,7 | * | 0,26 |
| $O_2$ | 2,9 | 4,8 | 2,9 | 7,0 | * | 0,02 |
| Ar | 2,2 | 3,7 | 2,2 | 5,3 | * | 0,01 |
| $N_2$ | 0,8 | 1,3 | 0,8 | 1,9 | * | 0,01 |
|  | 60,0 | | 41,5 | | 18,5* | |

* Differenz (18,5-18,4 Nl/h) ist die Summe von $CH_4$, $CO_2$, $O_2$, Ar und $N_2$.

Es werden ca. 95 % des im Einsatzgas enthaltenen Ethylens zurückgewonnen.

Beispiel 2

Benutzt wird eine Umlaufapparatur, bei der unter Druck absorbiert werden kann. Desorbiert wird bei einem Überdruck von 2 bar. Verwendet wird eine Silbertrifluoracetat-Lösung mit 530 g $CF_3COOAg/l$. Im folgenden sind die Versuchsdaten zusammengefaßt.

| Absorber | 14 bar  T = 20 °C |
| Desorber | 3 bar  T = 80 °C |
| Gas zum Absorber | 800 Nl/h |
| Umlauflösung | 6 l |

4

| | Eingang | | Abgas | | Reinethylen | |
| --- | --- | --- | --- | --- | --- | --- |
| | Nl/h | Vol.-% | Nl/h | Vol-% | Nl/h | Vol-% |
| CH$_4$ | 410,4 | 51,3 | 409,1 | 70,9 | 1,3 | 0,6 |
| C$_2$H$_4$ | 242,4 | 30,3 | 21,6 | 3,7 | 220,8 | 99,0 |
| CO$_2$ | 33,6 | 4,2 | 32,9 | 5,7 | 0,7 | 0,3 |
| O$_2$ | 38,4 | 4,8 | 38,2 | 6,7 | 0,2 | 0,1 |
| Ar | 64,0 | 8,0 | 64,0 | 11,1 | — | < 0,1 |
| N$_2$ | 11,2 | 1,4 | 11,2 | 1,9 | — | < 0,1 |
| | 800 | | 577 | | 223 | |

Es werden ca. 91 % des im Einsatzgas enthaltenen Ethylens zurückgewonnen.

## Beispiel 3

Benutzt wird eine Umlaufapparatur, bei der unter geringem Überdruck absorbiert wird. Die Desorption erfolgt bei Atmosphärendruck. Der Absorber wird mit einem propylenhaltigen Gasstrom beaufschlagt, Die verwendete Silbertrifluoracetatlösung enthält 600 g CF$_3$COOAg/l. Die Versuchsdaten sind im folgenden zusammengefaßt.

| Absorber | 1,25 bar T = 20 °C |
| --- | --- |
| Desorber | 1 bar T = 80 °C |
| Gas zum Absorber | 60 Nl/h |
| Umlauflösung | 1,2 l/h |

| | Eingang | | Abgas | | Reinpropylen | |
| --- | --- | --- | --- | --- | --- | --- |
| | Nl/h | Vol-% | Nl/h | Vol-% | Nl/h | Vol-% |
| N$_2$ | 34,1 | 56,8 | 33,7 | 94,9 | 0,4 | 1,6 |
| C$_3$H$_6$ | 24,6 | 41,1 | 0,5 | 1,4 | 24,1 | 98,4 |
| C$_3$H$_8$ | 1,3 | 2,1 | 1,3 | 3,7 | — | 0,01 |
| | 60,0 | | 35,5 | | 24,5 | |

Es werden ca. 98 % des im Einsatz enthaltenen Propylens zurückgewonnen.

## Ansprüche

1. Verfahren zur Gewinnung von Ethylen aus ethylenhaltigen Gasen durch Absorption des Ethylens in einer wäßrigen Silbersalz-Lösung und Rückgewinnung durch Desorption, dadurch gekennzeichnet, daß man ein 5-95 Vol.-% Ethylen enthaltendes Gas mit einer Lösung von Silbertrifluoracetat behandelt und die Absorption des Ethylens in an sich bekannter Weise bei höherem Druck und/oder niedrigerer Temperatur als die Desorption durchführt.

2. Verfahren nach Anspruch 1, dadurch gekennzeichnet, daß das Silbertrifluoracetat in der wäßrigen Absorptionslösung in einer Konzentration von 400-720 g CF$_3$COOAg/l vorliegt.

3. Verfahren nach Anspruch 1 und 2, dadurch gekennzeichnet, daß die Konzentration des Silbertrifluoracetats in der Absorptionslösung 500-660 g/l beträgt.

4. Verfahren nach Anspruch 1 bis 3, dadurch gekennzeichnet, daß das ethylenhaltige Gas noch gesättigte Kohlenwasserstoffe und/oder CO$_2$ und/oder CO und/oder O$_2$ und/oder H$_2$ und/oder N$_2$ und/oder Edelgase enthält.

5. Verfahren nach Anspruch 1 bis 4, dadurch gekennzeichnet, daß das ethylenhaltige Gas ein Abgas aus der Ethylenoxid-Herstellung ist.

6. Verfahren nach Anspruch 5, dadurch gekennzeichnet, daß das Abgas für den Fall, daß Methan als Inertgas-Verdünnung bei der Ethylenoxid-Herstellung eingesetzt wird, folgende Zusammensetzung hat : 35-55 Vol.-% Methan, 20-35 Vol.-% Ethylen, 4-6 Vol.-% CO$_2$, 3-6 Vol.-% O$_2$, 0,1-10 Vol.-% Argon und 0,1-10 Vol.-% N$_2$.

7. Verfahren nach Anspruch 5, dadurch gekennzeichnet, daß das Abgas für den Fall, daß Stickstoff als Inertgas-Verdünnung bei der Ethylenoxid-Herstellung eingesetzt wird, folgende Zusammensetzung hat : 20-35 Vol.-% Ethylen, 4-6 Vol.-% CO$_2$, 3-6 Vol.-% O$_2$, 0,1-10 Vol.-% Argon und 35-60 Vol.-% N$_2$.

8. Verfahren nach Anspruch 1 bis 7, dadurch gekennzeichnet, daß die Absorption bei 10-60 °C und 1-25 bar und die Desorption bei 15-100 °C und 0,05-4 bar durchgeführt werden.

9. Verfahren nach Anspruch 1 bis 8, dadurch gekennzeichnet, daß die Absorption des Ethylens bei einem Druck von mehr als 2 bar durchgeführt wird.

10. Verfahren nach Anspruch 1 bis 9, dadurch gekennzeichnet, daß man in kontinuierlicher Arbeitsweise die Absorption bei 15-30 °C und einem Druck von mindestens 2 bar und die Desorption bei 50-90 °C und einem Druck von mindestens Normaldruck durchführt, wobei der Druck für die Absorption höher ist als der Druck für die Desorption.

## Claims

1. Process for obtaining ethylene from ethylene-containing gases by absorption of the ethylene in an aqueous silver salt solution and recovery of the ethylene by desorption, characterised in that a gas containing 5-95 % by volume of ethylene is treated with a solution of silver trifluoroacetate and the absorption of the ethyle is conducted in a manner know per se under a higher pressure and/or at a lower temperature than the desorption.

2. Process according to Claim 1, characterized in that the silver trifluoroacetate is present in the aqueous absorption solution in a concentration of 400-720 g of $CF_3COOAg/l$.

3. Process according to Claim 1 and 2, characterised in that the concentration of the silver trifluoroacetate in the absorption solution is 500-660 g/l.

4. Process according to Claim 1 to 3, characterised in that the ethylene-containing gas also contains saturated hydrocarbons and/or $CO_2$ and/or CO and/or $O_2$ and/or $H_2$ and/or $N_2$ and/or noble gases.

5. Process according to Claim 1 to 4, characterised in that the ethylene-containing gas is an off-gas from the preparation of ethylene oxide.

6. Process according to Claim 5, characterised in that the off-gas has, in the case where methane is used as the inert gas diluent in the preparation of ehtylene oxide, the following composition : 35-55 % by volume of methane, 20-35 % by volume of ethylene, 4-6 % by volume of $CO_2$, 3-6 % by volume of $O_2$, 0.1-10 % by volume of argon and 0.1-10 % by volume of $N_2$.

7. Process according to Claim 5, characterised in that the off-gas has, in the case where nitrogen is used as the inert gas diluent in the preparation of ethylene oxide, the following composition : 20-35 % by volume of ethylene, 4-6 % by volume of $CO_2$, 3-6 % by volume of $O_2$, 0.1-10 % by volume of argon and 35-60 % by volume of $N_2$.

8. Process according to Claim 1 to 7, characterised in that the absorption is carried out at 10-60 °C and under 1-25 bars and the desorption is carried out at 15-100 °C and under 0.05-4 bars.

9. Process according to Claim 1 to 8, characterised in that the absorption of the ethylene is carried out under a pressure of more than 2 bars.

10. Process according to Claim 1 to 9, characterised in that in a continuous method of procedure the absorption is carried out at 15-30 °C and under a pressure of at least 2 bars and the desorption is carried out at 50-90 °C and under a pressure of at least normal pressure, the pressure for the absorption being higher than the pressure for the desorption.

## Revendications

1. Procédé pour obtenir de l'éthylène à partir de gaz contenant de l'éthylène, par absorption de l'éthylène dans une solution aqueuse d'un sel d'argent et récupération par désorption, caractérisé en ce qu'on traite un gaz contenant 5 à 95 % en volume d'éthylène par une solution de trifluoracétate d'argent et en ce qu'on effectue l'absorption de l'éthylène, de façon connue en soi, à une pression supérieure et/ou à une température inférieure à celles de la désorption.

2. Procédé selon la revendication 1, caractérisé en ce que le trifluoracétate d'argent se trouve dans la solution aqueuse d'absorption en une concentration de 400 à 720 g de $CF_3COOAg/l$.

3. Procédé selon l'une des revendications 1 et 2, caractérisé en ce que la concentration du trifluoracétate d'argent dans la solution d'absorption se situe entre 500 et 660 g/l.

4. Procédé selon l'une des revendications 1 à 3, caractérisé en ce que le gaz contenant de l'éthylène contient également des hydrocarbures saturés et/ou $CO_2$ et/ou $O_2$ et/ou $H_2$ et/ou $N_2$ et/ou des gaz rares.

5. Procédé selon l'une des revendications 1 à 4, caractérisé en ce que le gaz contenant de l'éthylène est un gaz provenant de la production de l'oxyde d'éthylène.

6. Procédé selon la revendication 5, caractérisé en ce que, si le méthane est utilisé comme gaz inerte de dilution lors de la production de l'oxyde d'éthylène, le gaz qui en provient présente la composition suivante : 35 à 55 % en volume méthane, 20 à 35 % en volume d'éthylène, 4 à 6 % en volume de $CO_2$, 3 à 6 % en volume de $O_2$, 0,1 à 10 % en volume d'argent et 0,1 à 10 % en volume de $N_2$.

7. Procédé selon la revendication 5, caractérisé en ce que, si l'azote est utilisé comme gaz inerte de dilution lors de la production de l'oxyde d'éthylène, le gaz qui en provient présente la composition suivante : 20 à 35 % en volume d'éthylène, 4 à 6 % en volume de $CO_2$, 3 à 6 % en volume de $O_2$, 0,1 à 10 % en volume d'argent et 35 à 60 % en volume de $N_2$.

8. Procédé selon l'une des revendications 1 à 7, caractérisé en ce qu'on effectue l'absorption à 10-60 °C et 1-25 bars et la désorption à 15-100 °C et 0,05-4 bars.

9. Procédé selon l'une des revendications 1 à 8, caractérisé en ce qu'on effectue l'absorption de l'éthylène sous une pression supérieure à 2 bars.

10. Procédé selon l'une des revendications 1 à 9, caractérisé en ce que, dans un mode opératoire continu, on effectue l'absorption à 15-30 °C et sous une pression d'au moins 2 bars, et la désorption à 50-90 °C et sous une pression qui est au moins la pression normale, de sorte que la pression pour l'absorption est supérieure à la pression pour la désorption.

FIG. 1